# EUROPEAN PATENT APPLICATION

(11) **EP 2 454 945 A1**
(43) Date of publication of application: **23.05.2012**
(21) Application number: 10190957.0
(22) Date of filing: 12.11.2010
(51) Int. Cl.: A23F 5/02, A61K 31/216, A61K 36/185

(54) **Methods of improving mental or physical health conditions in an individual**

(71) Applicant: Nestec S.A., 1800 Vevey (CH)
(72) Inventor: Silber, Yvonne Beata, 1072 Forel (CH); Schmitt, Jeroen Antonius Johannes, 1510 Moudon (CH)
(74) Representative: Lomholt, Stig Bredsted

(57) **Abstract**

Methods of improving mental or physical health conditions of an individual are provided. In a general embodiment, the method provides administering to an individual in need of an improved mental or physical health condition a decaffeinated coffee product comprising an unroasted coffee. The mental or physical health condition can be, for example, sustained attention in the individual, improvement in the individual's mood or alertness, reduced headaches in the individual, and/or improved relaxation in the individual.

## Description

The present disclosure generally relates to methods for improving mental or physical health conditions of an individual. More specifically, the present disclosure relates to methods of improving the attention, mood, alertness and/or relaxation or reducing headaches of an individual.

Coffee is a complex mixture of many hundreds of compounds, which, in combination, form a unique and pleasing aroma and taste desired by many consumers. Furthermore, coffee is consumed not only for its desirable flavor but often for other reasons, such as to increase short term mental alertness. The positive health impact of coffee has been studied over many decades and, for a long time, it has been known that certain coffee compounds are capable of providing benefits to the consumer, especially increased mental alertness through the ingestion of caffeine. However, it is less well known to consumers that certain coffee compounds are excellent anti-oxidants and that, weight for weight, coffee can potentially provide significantly more antioxidants to the consumer than, for example, a well known source of antioxidants such as green tea.

A very important source of antioxidants that has been identified in coffee is the class known as chlorogenic acids. Chlorogenic acids ("CGA") in coffee are mainly mono- and di-esters of quinic acid and phenolic groups (e.g., caffeic, ferulic, coumaric, methoxycinnamic) attached to different positions. In vitro studies have confirmed the antioxidant properties of CGA, which prevents oxidation by chelating metals and scavenging reactive oxygen species. In addition to its antioxidant effects, rodent models have also shown CGA compounds to have anxiolytic, anti-inflammatory, analgesic and antipyretic effects, weak caffeine-like psychostimulant actions on spontaneous locomotor activity. A distinct neuroprotective action of CGA compounds is also evidenced by its ability to protect neuronal cells from beta-amyloid-induced toxicity and oxidative stress.

From the health and nutritional perspective, it is desirable that consumers should be able to benefit from the positive health aspects of coffee identified above and so it would be highly advantageous to utilize the beneficial components of coffee such as chlorogenic acids. However, few studies have been performed to show the impact these beneficial components on a variety of mental and physical health conditions of an individual.

### SUMMARY

The present disclosure provides methods for improving the mental or physical health conditions in an individual. In an embodiment, the present disclosure provides a method of sustaining attention in an individual. The method comprises administering to an individual in need of sustained attention a decaffeinated coffee product including an unroasted coffee to sustain attention in the individual. The decaffeinated coffee product can have a similar effect in the individual as an approximately equivalent amount of a caffeinated coffee product.

In another embodiment, the present disclosure provides a method of improving an individual's mood or alertness. The method comprises administering to an individual in need improved mood or alertness a decaffeinated coffee product including an unroasted coffee to improve the mood or alertness in the individual. The decaffeinated coffee product can have a similar effect in the individual as an approximately equivalent amount of a caffeinated coffee product.

In an alternative embodiment, the present disclosure provides a method of improving relaxation in an individual. The method comprises administering to an individual in need of relaxation a decaffeinated coffee product including an unroasted coffee to improve relaxation in the individual. The decaffeinated coffee product can have a similar effect in the individual as an approximately equivalent amount of a caffeinated coffee product.

In another embodiment, the present disclosure provides a method of reducing a headache in an individual. The method comprises administering to an individual having a headache a decaffeinated coffee product including an unroasted coffee to reduce the headache in the individual. The decaffeinated coffee product can have a similar effect in the individual as an approximately equivalent amount of a caffeinated coffee product.

In any of the methods described herein, the decaffeinated coffee product includes a mixture of unroasted coffee and roasted coffee. In any of the methods described herein, the unroasted coffee is in the form of an extract of an unroasted coffee. For example, the extract can be derived from (i) a first portion including unroasted ground and/or unground coffee, in an amount of from 1 to 90% by weight based on the total weight of the coffee product, and (ii) a second portion including ground coffee that has been roasted to a higher degree of roast than the first portion, in an amount of from 99 to 10% by weight based on the total weight of the coffee product, wherein the content of the chlorogenic acids is at least 4 g per 100 g of the coffee product.

In yet another embodiment, the present disclosure provides a method of sustaining attention in an individual. The method comprises administering to an individual in need of sustained attention a non-coffee product including an unroasted coffee extract and/or chlorogenic acids to sustain attention in the individual.

In still another embodiment, the present disclosure provides a method of improving an individual's mood or alertness. The method comprises administering to an individual in need of an improved mood or alertness a non-coffee product including an unroasted coffee extract and/or chlorogenic acids to improve the mood or alertness of the individual.

In another embodiment, the present disclosure provides a method of improving relaxation in an individual. The method comprises administering to an individual in need of an improved mood or alertness a non-coffee product including an unroasted coffee extract and/or chlorogenic acids to improve the relaxation of the individual.

In yet another embodiment, the present disclosure provides a method of reducing a headache in an individual. The method comprises administering to an individual having a headache a non-coffee product including an unroasted coffee extract and/or chlorogenic acids to reduce the headache of the individual.

In any of the methods herein, the chlorogenic acids can be derived from a food source (including coffee). The non-coffee product can be a food product, a beverage, a food supplement, a pet care product, a pharmaceutical product or a combination thereof.

In an alternative embodiment, the present disclosure provides a method of making a consumable product for improving an individual's mental capabilities. The method comprises providing an unroasted coffee extract for improving the individual's mental capabilities, and adding the unroasted coffee extract to a consumable product. The consumable product can be a food product, a beverage, a food supplement, a pet care product, a pharmaceutical product or a combination thereof. The mental capabilities can be alertness, mood, attention or a combination thereof.

In another embodiment, the present disclosure provide a method of improving an individual's mental capabilities. The method comprises providing an extract from an unroasted coffee product, adding the extract to a consumable product, and administering to an individual in need thereof the consumable product including the extract. The consumable product can be a food product, a beverage, a food supplement, a pet care product, a pharmaceutical product or a combination thereof. The mental capabilities can be alertness, mood, attention or a combination thereof.

In still another embodiment, the present disclosure provides a method of improving an individual's mental capabilities. The method comprises administering to an individual in need thereof a consumable product including chlorogenic acids at a rate of about 1 to about 150 mg chlorogenic acids/kg bodyweight per day. In yet another embodiment, the present disclosure provides a method of improving an individual's mental capabilities. The method comprises administering to an individual in need thereof a decaffeinated coffee product including chlorogenic acids at a rate of about 1 to about 150 mg chlorogenic acids/kg bodyweight per day.

In an embodiment, the present disclosure provides the use of an unroasted coffee extract to improve the attention, mood, relaxation or alertness or reduce the headache of an individual. In another embodiment, the present disclosure provides the use of chlorogenic acids to improve the attention, mood, relaxation or alertness or reduce the headache of an individual.

In another embodiment, the present disclosure provides a method of increasing sports performance in an individual. The method comprises administering to an individual in need of thereof a decaffeinated coffee product comprising an unroasted coffee to increase the sports performance in the individual. In an alternative embodiment, the present disclosure provides a method of increasing sports performance in an individual. The method comprises administering to an individual in need of thereof a non-coffee product comprising a component selected from the group consisting of an unroasted coffee extract, chlorogenic acids and combinations thereof to increase the sports performance in the individual.

An advantage of the present disclosure is to provide a method of improving a mental health condition of an individual using a decaffeinated coffee product including an unroasted coffee.

Another advantage of the present disclosure is to provide a method of improving a physical health condition of an individual using a decaffeinated coffee product including an unroasted coffee.

Still another advantage of the present disclosure is to provide a method of improving a mental or physical health condition of an individual using chlorogenic acids.

Yet another advantage of the present disclosure is to provide a method of improving the attention, mood, alertness and/or relaxation or reducing headaches of an individual using an extract from a coffee product.

Additional features and advantages are described herein, and will be apparent from, the following Detailed Description and the figures.

### BRIEF DESCRIPTION OF THE FIGURES

FIG. 1 shows median accuracy difference scores (time 2 minus time 1) are shown for the Rapid Visual Information processing task ("RVIP"), which is a measure of sustained attention. The p-values are presented before correction for multiple testing.

FIG. 2 shows mean difference scores (with SEM error bars) of Caffeine Research Visual Analogue Scale ("CRVAS") mood items for each treatment condition.

FIG. 3 shows mean difference scores (with SEM error bars) for the Bond-Lader mood dimension "Alert" for each treatment condition.

FIG. 4 shows mean emotion face recognition task negative bias accuracy index difference score (with SEM error bars) for each treatment condition.

### DETAILED DESCRIPTION

The present disclosure relates generally to methods for improving the mental and/or physical health conditions individual. In a general embodiment, the method provides administering to an individual in need of an improved mental or physical health condition a decaffeinated coffee product including an unroasted coffee (e.g., green coffee beans). The decaffeinated coffee product can include portions of an unroasted and a roasted coffee in any suitable form.

Without being bound by theory, it has been surprisingly found that providing individuals with components of an unroasted coffee (e.g., via an extract) can improve various mental and physical health conditions in an individual. The mental or physical health condition can be, for example, sustained attention in the individual, improvement in the individual's mood or alertness, reduced headaches in the individual, and/or improved relaxation in the individual. These improved mental or physical health conditions can include those not normally attributable to caffeine typically found in coffee products. It should be noted that an effect on recovery from mental fatigue is different from effects on functions such as alertness and attention, and it is not automatic that a compound having an effect on recovery from mental fatigue will have an effect on the latter functions.

As used herein, the term "attention" refers to the ability to direct and focus cognitive activity on specific stimuli. It is the ability or power to concentrate mentally. Sustained attention refers to the state in which attention must be maintained over time.

As used herein, the term "mental alertness" refers to the state of paying close and continuous attention, being watchful and prompt, or being quick to perceive and/or act. It is being vigilantly attentive, mentally responsive and perceptive, and quick.

As used herein, the term "mental fatigue" refers to a state of awareness describing a range of afflictions, associated with mental weakness. Such mental fatigue can manifest itself either as somnolence (decreased wakefulness) or as a general decrease of attention (not necessarily sleepiness). It may also be described as a decreased level of consciousness. Mental fatigue can be caused by continual mental effort and attention on a particular task, as well as high levels of stress or emotion. Basically, any mental process that goes into overload can result in mental fatigue.

As used herein, the term "mood" refers to a state or quality of feeling (an emotional state) at a particular time. Moods differ from simple emotions in that they are less specific, less intense, and less likely to be triggered by a particular stimulus or event. Moods generally have either a positive or negative valence. Mood is an internal, subjective state.

As used herein, the term "relaxation" refers to the act of mentally relaxing or the state of being mentally relaxed.

As used herein, the term "headache" refers to a persistent or lasting pain or discomfort in the head region.

As used herein, the term "sports performance" refers to an endurance sport, sustained high-intensity sport or team sports all requiring physical performance.

As used herein, the term "decaffeinated coffee product" refers to a coffee product that has some, most or all of the caffeine removed (e.g., via a decaffeination process). For example, the coffee product can have a reduced amount of caffeine (e.g., 95% removal, 97% removal, 99% removal) compared to the amount normally associated with that coffee product.

It has been postulated, that chlorogenic acids can assist in improving various mental or physical health conditions of the individual. Chlorogenic acids have the general formula:

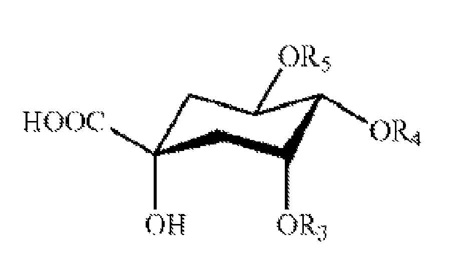

The most prevalent chlorogenic acids are given in the following table.

| Name | Structure Chlorogenic acids | R3 | R4 | R5 |
|---|---|---|---|---|
| 5-O-caffeoyl-D-quinic acid | C | H | H | caffeoyl |
| (5CQA) | | | | |
| 4-O-caffeoyl-D-quinic acid | C | H | caffeoyl | H |
| (4CQA) | | | | |
| 3-O-caffeoyl-D-quinic acid | C | caffeoyl | H | H |
| (3CQA) | | | | |

Other isomers exist which also fall within the definition of the structure given above. Therefore, in the context of the present disclosure, the phrase "chlorogenic acids" denotes all chlorogenic acid isomers, which are defined by the aforementioned structure.

In an embodiment, the present disclosure provides a method of sustaining attention in an individual. The method comprises administering to an individual in need of sustained attention a decaffeinated coffee product including an unroasted coffee to sustain attention in the individual. The decaffeinated coffee product can be partially or fully decaffeinated. The decaffeinated coffee product can have a similar effect in the individual as an approximately equivalent amount of a caffeinated coffee product.

In another embodiment, the present disclosure provides a method of improving an individual's mood or alertness. The method comprises administering to an individual in need improved mood or alertness a decaffeinated coffee product including an unroasted coffee to improve the mood or alertness in the individual. The decaffeinated coffee product can be partially or fully decaffeinated. The decaffeinated coffee product can have a similar effect in the individual as an approximately equivalent amount of a caffeinated coffee product.

In an alternative embodiment, the present disclosure provides a method of improving relaxation in an individual. The method comprises administering to an individual in need of relaxation a decaffeinated coffee product including an unroasted coffee to improve relaxation in the individual. The decaffeinated coffee product can be partially or fully decaffeinated. The decaffeinated coffee product can have a similar effect in the individual as an approximately equivalent amount of a caffeinated coffee product.

In another embodiment, the present disclosure provides a method of reducing a headache in an individual. The method comprises administering to an individual having a headache a decaffeinated coffee product including an unroasted coffee to reduce the headache in the individual. The decaffeinated coffee product can be partially or fully decaffeinated. The decaffeinated coffee product can have a similar effect in the individual as an approximately equivalent amount of a caffeinated coffee product.

The following discussion of the decaffeinated coffee products and extracts applies to any embodiments of the present disclosure. In an embodiment, the decaffeinated coffee product can include a mixture of unroasted coffee and roasted coffee. In another embodiment, the decaffeinated coffee product can include a high content of chlorogenic acids or an amount of chlorogenic acids beyond the amount naturally associated with a roasted coffee product. For example, the decaffeinated coffee product including a high content of chlorogenic acids can be achieved by having a green and roasted coffee co-extraction (e.g., by having a proportion of green beans (i.e., not roasted) in the coffee blend). In an alternative embodiment, the decaffeinated coffee product can include an amount of chlorogenic acids ranging from about 100 mg to about 10,000 mg including for example, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1,000 mg, 2,000 mg, 3,000 mg, 4,000 mg, 5,000 mg, 6,000 mg, 7,000 mg, 8,000 mg, 9,000 mg and the like and any amount therebetween.

In another embodiment, the unroasted coffee is in the form of an extract. The extract can be derived from an unroasted coffee (e.g., green coffee beans) or a mixture of unroasted coffee and roasted coffee (e.g., beans). Details of such an extract can be found, for example, in U.S. Patent Serial No. 11/911,046, which is incorporated herein by reference. In an embodiment, the extract can be derived from (i) a first portion including unroasted ground and/or unground coffee, in an amount of from 1 to 90% by weight based on the total weight of the coffee product, and (ii) a second portion including ground coffee that has been roasted to a higher degree of roast than the first portion, in an amount of from 99 to 10% by weight based on the total weight of the coffee product, wherein the content of the chlorogenic acids is at least 4 g per 100 g of the coffee product.

In any embodiments of the present disclosure, the extract can be derived from a mixture of green (e.g., unroasted) and roasted coffee beans in which the weight ratio of the green portion and roasted portion in the coffee product is from 1:99 to 90:10, alternatively from 30:70 to 50:50, alternatively from 35:65 to 45:55 or alternatively from 37:63 to 42:58.

In an embodiment, the green coffee portion is substantially or wholly derived from Robusta coffee beans. In another embodiment, at least 65%, more preferably 75%, even more preferably 85%, most preferably 95%, for example 100% by weight of the green coffee beans are Robusta. This is because it has been found that Robusta coffee beans provide a greater level of chlorogenic acid per gram of bean than Arabica beans.

The roasted coffee portion may be a blend of Arabica and Robusta, although, in a preferred embodiment, the roasted portion is substantially or wholly derived from Arabica beans. This is because Arabica provides a richer taste and aroma profile, which is associated with coffee of higher quality.

The green coffee portion and/or the roasted coffee portion can partially or fully decaffeinated. This is to provide products the caffeine level of which can be modulated from caffeine-free to regular caffeine level. However, it is particularly preferred that the green coffee portion is from decaffeinated coffee. Preferably the coffee product is used to provide a soluble coffee product.

The coffee beans to be extracted may be whole or ground. In an embodiment, green coffee beans are co-extracted with roasted coffee beans, i.e., green and roasted coffee beans are extracted simultaneously in the same extraction system to yield a mixed extract. The most volatile aroma components can be stripped from the beans before extraction, for example, if the extract is to be used for the production of pure soluble coffee. Methods for stripping of volatile aroma components are well known in the art, for example, from EP 1078576, which is incorporated herein by reference.

Following stripping, the less volatile components are then subjected to an "extraction" step. Extraction is a term common in the art of processing soluble coffee and indicates a process where water and/or steam are used to extract a complex mixture of coffee components from the roasted, ground coffee bean.

Extraction of coffee beans can be done using any suitable extraction method known to the skilled artisan. For example, extraction can be performed using water and/or steam as described in EP 0916267, which is incorporated herein by reference. In an embodiment, the coffee beans may be extracted by any suitable method yielding an extract including a desired amount of chlorogenic acids.

After extraction, the product may undergo a concentration step, following which, if a stripping step has taken place, the stripped aroma can be reintroduced into the resulting extract. Finally, the aromatized extract can be dried according to any standard procedure, such as freeze-drying, spray-drying or agglomerating. This produces a solid soluble product that may suitably be in the form of a powder or granules. If the extract has been dried, it may be resuspended as necessary for administration.

The soluble coffee mixture may be produced by co-extraction of the green portion and the roasted portion. Alternatively, the soluble coffee product can be produced by separately providing a roasted coffee portion and a green coffee portion and then extracting them individually before combining the resultant extracts. This is advantageous as the extraction conditions can be adapted to suit roasted and unroasted beans respectively.

The coffee extract may undergo any suitable treatment to remove undesired components of the extract. The extract may be separated from the extracted coffee beans by any suitable method, for example, filtration or centrifugation. The separation may be performed to the extent practically and economically feasible and needed in view of the desired use of the extract. The separation may thus not be 100% complete, for example, a minor part of undissolved material from the beans may still be present with the extract after separation.

In yet another embodiment, the present disclosure provides a method of sustaining attention in an individual. The method comprises administering to an individual in need of sustained attention a non-coffee product including an unroasted coffee extract and/or chlorogenic acids to sustain attention in the individual.

In still another embodiment, the present disclosure provides a method of improving an individual's mood or alertness. The method comprises administering to an individual in need of an improved mood or alertness a non-coffee product including an unroasted coffee extract and/or chlorogenic acids to improve the mood or alertness of the individual.

In still another embodiment, the present disclosure provides a method of improving relaxation in an individual. The method comprises administering to an individual in need of an improved mood or alertness a non-coffee product including an unroasted coffee extract and/or chlorogenic acids extract to improve the relaxation of the individual.

In yet another embodiment, the present disclosure provides a method of reducing a headache in an individual. The method comprises administering to an individual having a headache a non-coffee product including an unroasted coffee extract and/or chlorogenic acids to reduce the headache of the individual.

In any of the methods described herein, the chlorogenic acids can be derived, for example, from any suitable food source including coffee, tea, blackberry, potato or tomato. The chlorogenic acids can be derived, for example, from any suitable non-food source such as chemical synthesis. In an embodiment, the amount of chlorogenic acids added to the non-coffee product ranges from about 100 mg to about 10,000 mg including for example, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1,000 mg, 2,000 mg, 3,000 mg, 4,000 mg, 5,000 mg, 6,000 mg, 7,000 mg, 8,000 mg, 9,000 mg and the like and any amount therebetween. The non-coffee product can be a food product, a beverage, a food supplement, a pet care product, a pharmaceutical product or a combination thereof.

In an alternative embodiment, the present disclosure provides a method of making a consumable product for improving an individual's mental capabilities. The method comprises providing an unroasted coffee extract for improving the individual's mental capabilities, and adding the unroasted coffee extract to a consumable product. The unroasted coffee extract may include an effective amount of chlorogenic acids for improving the individual's mental capabilities. The consumable product can be a food product, a beverage, a food supplement, a pet care product, a pharmaceutical product or a combination thereof. The mental capabilities can be alertness, mood, attention or a combination thereof.

In any embodiments of the present disclosure, the unroasted coffee extract can be used separately from the extracted coffee beans. The undissolved material from the beans can be substantially removed by separation as described herein and is not used in the production of the food or beverage product.

The unroasted coffee extract and/or chlorogenic acids can be added as an ingredient in any suitable form (e.g., liquid, powder, granules, etc.) to any suitable consumable product. In an embodiment, the consumable product may be any food or beverage product known in the art. For example, the food or beverage products can be a coffee beverage, pure soluble coffee, a soft drink, a dietary supplement, a dairy product, a cereal product, a fruit or vegetable juice product, or a confectionary product, such as a chocolate product, for example a chocolate drink. A soluble coffee product may be produced by concentrating and drying the unroasted coffee extract. A soluble coffee product produced from the unroasted coffee extract may be sold as such, or may, for example, be mixed with a creamer and/or sweetener and sold to prepare a coffee beverage including creamer and/or sweetener, for example, cappuccino or cafe latte.

In another embodiment, the present disclosure provide a method of improving an individual's mental capabilities. The method comprises providing an extract from an unroasted coffee product, adding the extract to a consumable product, and administering to an individual in need thereof the consumable product including the extract. The consumable product can be a food product, a beverage, a food supplement, a pet care product, a pharmaceutical product or a combination thereof. The mental capabilities can be alertness, mood, attention or a combination thereof.

In still another embodiment, the present disclosure provides a method of improving an individual's mental capabilities. The method comprises administering to an individual in need thereof a consumable product including chlorogenic acids at a rate of about 1 to about 150 mg chlorogenic acids/kg bodyweight per day. In yet another embodiment, the present disclosure provides a method of improving an individual's mental capabilities. The method comprises administering to an individual in need thereof a decaffeinated coffee product including chlorogenic acids at a rate of about 1 to about 150 mg chlorogenic acids/kg bodyweight per day.

In an embodiment, the present disclosure provides the use of an unroasted coffee extract to improve the attention, mood, relaxation or alertness of an individual. In another embodiment, the present disclosure provides the use of an unroasted coffee extract to reduce the headache of an individual. In still another embodiment, the present disclosure provides the use of an effective amount of chlorogenic acids to improve the attention, mood, relaxation or alertness of an individual. In yet another embodiment, the present disclosure provides the use of an effective amount of chlorogenic acids to reduce the headache of an individual.

In another embodiment, the present disclosure provides a method of increasing sports performance in an individual. The method comprises administering to an individual in need of thereof a decaffeinated coffee product comprising an unroasted coffee to increase the sports performance in the individual. In an alternative embodiment, the present disclosure provides a method of increasing sports performance in an individual. The method comprises administering to an individual in need of thereof a non-coffee product comprising a component selected from the group consisting of an unroasted coffee extract, chlorogenic acids and combinations thereof to increase the sports performance in the individual.

Caffeine is known to improve sports performance by improving attention, increasing alertness, and reducing mental fatigue (thus the improvement comes directly from improving cognitive function). Accordingly, improvements in cognitive processes as discussed herein are the same attributes that have been known to improve sports performance (e.g., alertness, sustained attention, reducing mental fatigue).

### EXAMPLES

By way of example and not limitation, the following examples are illustrative of various embodiments of the present disclosure.

### EXAMPLE 1

### Introduction

Caffeine exerts positive effects on cognitive and behavioral processes, especially in sub-optimal conditions when arousal state is low. However, apart from caffeine, coffee contains other compounds that have purported antioxidant properties. The potential beneficial cognitive effects of non-caffeine constituents in coffee are poorly understood. The chlorogenic acids ("CGA") are the most abundant family of compounds found in coffee. However, the effects of these polyphenols on cognition and mood have not been investigated.

To ascertain whether chlorogenic acid found in coffee modulates brain function, the present study examined the acute effects of regular caffeinated coffee, decaffeinated coffee with regular chlorogenic acid content, decaffeinated coffee with high chlorogenic acid content and placebo, on mood and cognitive processes, as measured by behavioral tasks and event-related potentials ("ERPs") from brain electrical activity. Performance and ERP responses to a battery of cognitive tasks were recorded at baseline and following the equivalent of 3 cups of coffee in a randomized, double-blind, crossover study of 39 healthy older participants (53-79 years). Although there was no effect of coffee on the ERP indices, the decaffeinated coffee high in chlorogenic acid (in addition to the caffeinated coffee), improved sustained attention and mood by specifically increasing alertness and feelings of relaxation and decreasing reports of headaches and feelings of mental fatigue. These results provide the first indication that non-caffeine compounds in coffee, such as the chlorogenic acids, are capable of exerting acute behavioral effects, and may contribute to the positive mood and attentional effects of coffee in the elderly.

### Methodology and Trials

Cognitive, mood and electrophysiological data were collected from 39 healthy older participants (50+) who were light to moderate coffee drinkers (i.e., drinking no more than 8 cups of coffee per week). A randomized, double blind, placebo-controlled cross-over-design was employed. Each participant was tested under four acute treatment conditions which contained differing amounts of CGA and caffeine, each separated by at least a one-week washout period: (1) 6 grams of a soluble decaffeinated coffee product containing regular (224 mg) total CGA content (regular CGA Decaf), (2) 6 grams of a specially processed soluble coffee co-extracted from roasted and green (unroasted) beans as disclosed in WO 2006/108578, which is incorporated herein by reference, with high (521 mg) total CGA content (high CGA Decaf), (3) 6 grams of a soluble caffeinated coffee containing moderate-high total caffeine (167 mg) and regular (244 mg) total CGA (Caffeine) and (4) 6 grams of Placebo, made of maltodextrin mixed with coffee flavor and color (0 mg CGA and 0 mg caffeine). Table 1 provides details of the coffee composition. Note that 6 grams of coffee is equivalent to 3 standard servings.

A selected battery of cognitive and event related potential tasks were performed to measure memory, attention, executive functioning, emotional attention, learning, early sensory processing and information processing speed (Table 2 provides a summary of complete battery of tasks).

**Table 1. Composition of the study coffee treatments per 1 gram**

| **Coffee compound** | **Regular CGA Decaf** | **High CGA Decaf** | **Caffeinated coffee** | **Placebo** |
|---|---|---|---|---|
| ***CGA & its derivatives*** | | | | |
| 3-cqa | 6.9 | 18.0 | 7.3 | 0 |
| 4-cqa | 7.4 | 18.3 | 8.2 | 0 |
| 5-cqa | 12.6 | 29.5 | 14.0 | 0 |
| *sum CQA's* | 26.9 | 65.8 | 29.5 | 0 |
| 3-fqa | 1.6 | 0.8 | 1.5 | 0 |
| 4-fqa | 2.0 | 3.9 | 2.1 | 0 |
| 5-fqa | 1.9 | 4.8 | 2.2 | 0 |
| *sum FQA's* | 5.6 | 9.6 | 5.8 | 0 |
| 3,4-dicqa | 0.5 | 3.1 | 0.7 | 0 |
| 3,5-dicqa | 0.3 | 2.0 | 0.4 | 0 |
| 4,5-dicqa | 0.5 | 3.1 | 0.7 | 0 |
| *sum diCQA's* | 1.3 | 8.2 | 1.7 | 0 |
| CQA lactones | 3.5 | 3.2 | 3.7 | 0 |
| **Total CGA's** | **37.30** | **86.83** | **40.71** | **0** |
| ***Caffeic acid*** | 0.2 | 0.4 | 0.3 | 0 |
| ***Ferulic acid*** | 0.02 | 0.03 | 0.03 | 0 |
| ***Caffeine*** | 0.8 | 1.9 | 27.9 | 0 |

| | | | | |
|---|---|---|---|---|
| CQA, caffeoylquinic acid; FQA, feruloylquinic acid; diCQA, dicaffeoylquinic acid; CGA, chlorogenic acids | | | | |

**Table 2. Neuropsychological Test Battery**

| **Test** | **Cognitive Domain** | **Outcome Measure** | **Reference** |
|---|---|---|---|
| Bond and Lader Visual Analogue Scales (VAS) (Bond and Lader, 1974) | Self-report mood | alertness, calmness, and contentedness mood factors | Bond and Lader (1974) |
| Caffeine Research Visual Analogue Scales (CRVAS) | Self-report mood | relaxed, alert, jittery, tired, tense, headache, overall mood, mentally fatigued scales | Kennedy and Scholey (2004); Rogers *et al* (2003) |
| Visual Verbal Learning Test (VVLT) immediate verbal recall (3 trials) | Short-term memory | maximum number of correctly recalled words (%) in any 3 trials | Evers *et al* (2005); Kennedy *et al* (2003) |
| Rapid Visual Information Processing (RVIP) | Sustained attention | Accuracy, mean RT, false alarms, P300, CNV | Gilbert *et al* (2007); Jones *et al* (1992) |
| Mismatch Negativity (MMN) | Pre-attentive functioning, auditory discrimination | MMN mismatch waveform¹ | Leung *et al* (2008); Näätänen, (1992) |
| VVLT verbal delayed recall | Retrieval from long-term memory | Number of correctly recalled words (%) | Evers *et al* (2005) |
| VVLT delayed recognition | Storage in long-term memory | reaction time and accuracy of original words as percentages | |
| Emotional Face Recognition Task | Emotional processing | N170, N250 and accuracy negative bias² | Labuschagne *et al* (2008) |
| Inspection Time (IT) | Perceptual speed | IT (ms) | Nathan *et al* (2004) |
| Stroop Color-Word Test (SCWT) (Hammes, 1973) | Cognitive flexibility, susceptibility to cognitive interference | Stroop Interference³ | Van der Elst *et al* (2006) |

| | | | |
|---|---|---|---|
| Tasks are listed in order of presentation. The cited references provide a basic description of the task. RT- reaction time; CNV- Contingent Negative Variation ¹Generated by a 'mismatch' process between the auditory input from the deviant stimulus with the auditory memory of the standard stimulus ²Negative emotional bias represents the tendency to preferentially process negative emotional stimuli. We defined negative bias as the difference between happy and sad values ³SCWT Interference represents the extra time needed to complete subtest III, relative to the average of subtest I and II (interference = time subtest III - (time subtest I + time subtest II) / 2) | | | |

### Results and Conclusions

In the following section, statistical significance are presented before correction for multiple testing.

### Rapid Visual Information Processing (RVIP) task results

Data could not be normally distributed, therefore non-parametric Wilcoxon's Sign Rank tests were conducted. Median and interquartile range and mean and standard deviation values for the accuracy index are presented in Table 3.

**Table 3. Medians (and IQRs) and Means (and SDs) are shown for the accuracy index of the primary analysis measure (Rapid Visual Information Processing task), for each of the treatment conditions and time points separately.**

| **Primary Measure** | **Treatment** | ***N*** | **Phase** | | | | | |
|---|---|---|---|---|---|---|---|---|
| | | | **Pre median (IQR)** | **Pre mean (SD)** | **Post median (IQR)** | **Post mean (SD)** | **Difference median (IQR)** | **Difference mean (SD)** |
| **Accuracy (%)** | ***High CGA decaf*** | 39 | 70.83 (29.17) | 65.4 (19.1) | 67.71 (28.65) | 66.2 (19.4) | 2.08 (8.33) | 0.9 (10.0) |
| | ***Caffeine*** | 39 | 67.71 (29.69) | 65.4 (20.5) | 72.92 (26.04) | 69.5 (18.9) | 3.12 (11.46) | 4.2 (9.5) |
| | ***Regular CGA decaf*** | 39 | 69.79 (23.44) | 67.1 (19.1) | 73.96 (31.77) | 67.7 (19.5) | 1.04(8.33) | 0.7(7.3) |
| | ***Placebo*** | 39 | 72.92 (32.81) | 66.4 (20.7) | 70.83 (30.73) | 64.4 (21.0) | -1.04 (11.98) | -2.0(9.8) |

Confirming that the experimental manipulation was achieved, caffeinated coffee substantially improved accuracy on the sustained attention task relative to Placebo (Z[39]=2.98, uncorrected p=0.003). That is, whereas accuracy reduced from baseline to Placebo, it increased from baseline to caffeine.

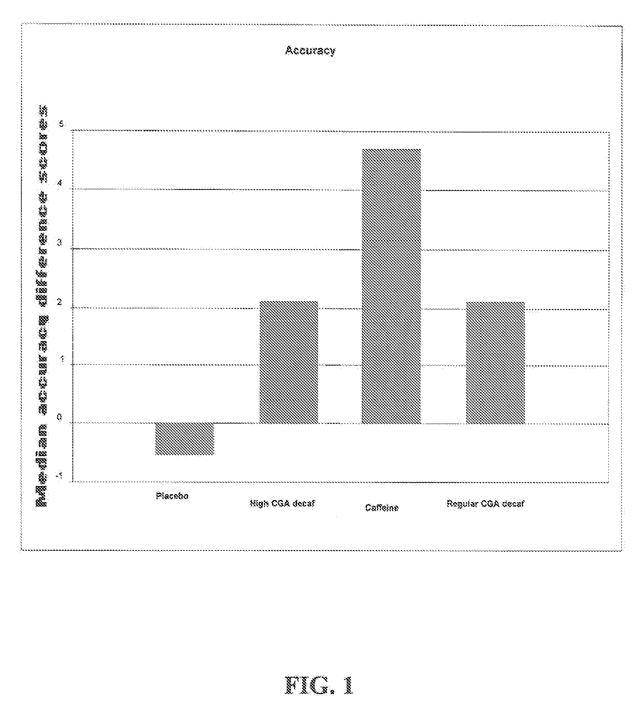

Relative to Placebo, high CGA content decaffeinated coffee improved Accuracy at a trend level (Z[39]=1.77; uncorrected p=0.077), where accuracy was reduced from baseline to post-treatment in the Placebo condition, and increased from baseline to post-treatment in the high CGA content decaffeinated coffee condition. However, no differences in accuracy were found between regular CGA content decaffeinated coffee and Placebo (Z[39]=1.18, uncorrected p=0.238). FIG. 1 illustrates the results. These results suggest that a high CGA content decaffeinated coffee may improve sustained attention.

### Mood results

Non-parametric analyses were conducted for each Bond and Lader Visual Analogue Mood Scale ("VAMS") item and Caffeine Research Visual Analogue Scale ("CRVAS") item separately. These were re-run with parametric tests, and results were unchanged. Table 4 presents the means and SDs for the mood outcome measures for each treatment by phase (baseline, post-treatment and difference score) separately.

**Table 4. Means (and SD) are shown for the mood measures for each of the treatment conditions and time points separately.**

| **Secondary Measure** | **Treatment** | ***N*** | **Phase** | | |
|---|---|---|---|---|---|
| | | | **Pre** | **Post** | **Diff** |
| **Accuracy 'Negative bias' (sad - happy)** | ***High CGA decaf*** | 39 | -27.1 (28.1) | -29.8 (27.0) | -2.7(16.8) |
| | ***Caffeine*** | 39 | -26.8 (22.2) | -27.4 (26.3) | -0.57 (19.0) |
| | ***Regular CGA decaf*** | 39 | -30.1 (23.3) | -24.9 (28.0) | 5.2(17.3) |
| | ***Placebo*** | 39 | -30.1 (22.1) | -30.4 (23.7) | -0.33 (13.25) |
| **Bond-Lader Alert** | ***High CGA decaf*** | 39 | 71.45 (18.67) | 72.26 (18.23) | 0.814(13.16) |
| | ***Caffeine*** | 38 | 73.17 (18.47) | 75.48 (18.48) | 2.31 (8.54) |
| | ***Regular CGA decaf*** | 38 | 75.96 (14.31) | 69.52 (18.84) | -6.43 (11.49) |
| | ***Placebo*** | 38 | 72.75 (17.17) | 69.52 (19.61) | -3.23 (11.32) |
| **CRVAS Relaxed** | ***High CGA decaf*** | 38 | 73.19 (19.75) | 74.37 (16.33) | 1.18 (14.18) |
| | ***Caffeine*** | 38 | 71.96 (16.82) | 76.73 (15.19) | 4.77 (10.20) |
| | ***Regular CGA decaf*** | 39 | 73.61 (15.73) | 70.59 (19.87) | -3.02 (19.03) |
| | ***Placebo*** | 39 | 75.05 (17.51) | 70.81 (18.13) | -4.25 (13.38) |
| **CRVAS Alert** | ***High CGA decaf*** | 38 | 66.28 (24.0) | 68.50 (20.58) | 2.22 (21.50) |
| | ***Caffeine*** | 38 | 67.05 (21.97) | 72.31 (19.13) | 5.26 (18.37) |
| | ***Regular CGA decaf*** | 39 | 70.67 (16.30) | 67.98 (20.71) | -2.70 (12.71) |
| | ***Placebo*** | 39 | 66.91 (22.13) | 62.87 (23.14) | -4.03 (22.93) |
| **CRVAS Tired** | ***High CGA decaf*** | 38 | 30.40 (22.81) | 35.62 (24.05) | 5.22 (23.49) |
| | ***Caffeine*** | 38 | 29.99 (23.78) | 28.87 (22.34) | -1.12 (25.29) |
| | ***Regular CGA decaf*** | 39 | 27.27 (18.33) | 38.70 (25.63) | 11.43 (18.43) |
| | ***Placebo*** | 39 | 28.37 (20.89) | 38.86 (24.83) | 10.50 (22.02) |
| **CRVAS Tense** | ***High CGA decaf*** | 38 | 18.61 (17.61) | 19.90 (17.45) | 1.29 (13.44) |
| | ***Caffeine*** | 38 | 19.05 (16.41) | 17.49 (18.47) | -1.56 (15.02) |
| | ***Regular CGA decaf*** | 39 | 17.68 (15.08) | 22.81 (19.85) | 5.13 (18.16) |
| | ***Placebo*** | 39 | 15.52 (14.49) | 20.22 (18.50) | 4.70 (15.26) |
| **CRVAS Headache** | ***High CGA decaf*** | 38 | 14.47 (27.52) | 12.34 (21.02) | -2.14 (31.37) |
| | ***Caffeine*** | 38 | 8.42 (11.61) | 7.73 (13.52) | -0.69 (10.7) |
| | ***Regular CGA decaf*** | 39 | 7.99 (12.13) | 13.51 (20.99) | 5.53 (17.99) |
| | ***Placebo*** | 39 | 8.09 (11.48) | 14.26 (19.84) | 6.17 (18.45) |
| **CRVAS Mentally Fatigued** | ***High CGA decaf*** | 38 | 32.73 (27.58) | 34.13 (24.87) | 1.40 (23.49) |
| | ***Caffeine*** | 38 | 34.81 (27.30) | 28.62 (22.40) | -6.20 (27.15) |
| | ***Regular CGA decaf*** | 39 | 25.53 (22.27) | 34.78 (27.50) | 9.24 (24.29) |
| | ***Placebo*** | 39 | 25.91 (22.64) | 39.21 (26.69) | 13.30 (22.75) |

For the CRVAS, consistent with previous findings, caffeinated coffee increased scores on items *Relaxed, Alert* and *Overall Mood,* and decreased scores on Items *Tired, Tense, Headache* and *Mentally Fatigued* relative to placebo (Z[38]=2.74, p=0.007, trend-level Z[38]=2.21, p=0.027, trend-level Z[38]=1.92, p=0.055, trend-level Z[38]=1.73, p=0.084, trend-level Z[38]=2.33, p=0.020, Z[38]=3.73, p<0.001, and Z[38]=3.46, p=0.001, respectively.

High CGA content decaffeinated coffee increased scores on items *Relaxed* and decreased scores on Items *Headache* and *Mentally Fatigued* relative to placebo (trend-level Z[38]=1.94, p=0.052, Z[38]=2.04, p=0.041, and trend-level Z[38]=1.94, p=0.052, respectively). There was no effect of regular CGA content decaffeinated coffee on any of the items except for trend-level increases in scores on *Headaches* and *Mentally Fatigued* relative to placebo (Z[38]=1.71, p=0.087 and Z[38]=1.76, p=0.079, respectively).

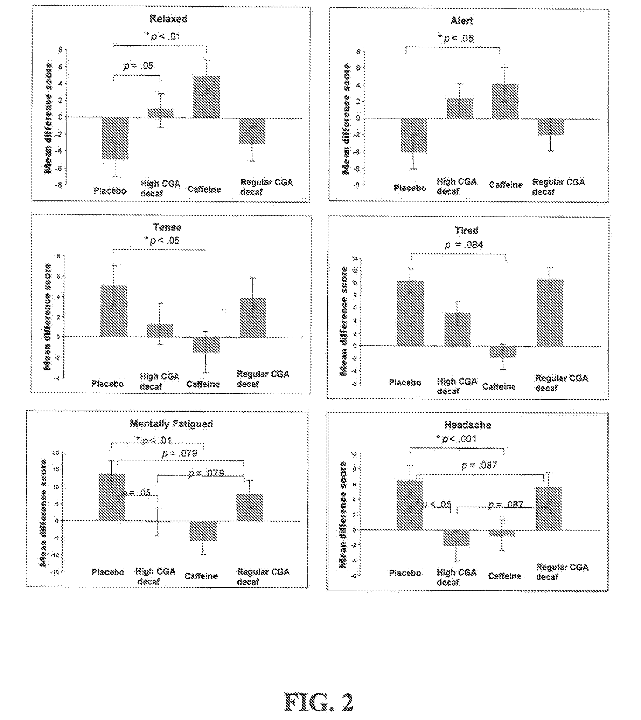

Further support for the modulating effects of high CGA content decaffeinated coffee on mood was manifested when compared to the low CGA content decaffeinated coffee. Specifically, high CGA content decaffeinated coffee showed trend-level decreases on report of *Headaches* and *Mentally Fatigued* relative to regular CGA decaffeinated coffee, which showed an increase over time (Z[38]=1.71, p=0.087, trend-level and Z[38]=1.78, p=0.074, trend-level, respectively). The results are depicted in FIG. 2.

Although not significant, the general direction of the results for the remainder of the CRVAS items, *Alert, Tired* and *Tense* followed a similar pattern (refer to FIG. 2). Specifically, the high CGA content decaffeinated coffee increased reports of alertness and decreased reports of tiredness and tension, which was significant for the caffeinated coffee, whereas the regular CGA content decaffeinated coffee and placebo had no beneficial effect.

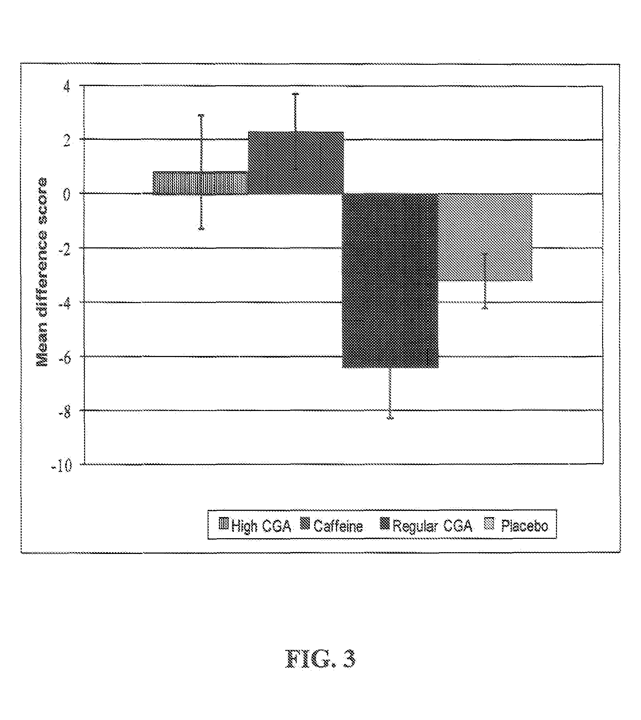

Consistent with these findings, for the Bond and Lader Visual Analogue Mood Scale test, high CGA content decaffeinated coffee and caffeinated coffee increased *Alertness* scores relative to placebo (trend-level Z(38)=1.86, p=0.062; Z[37]=2.04, p=0.042, respectively) and relative to the regular CGA content decaffeinated coffee (Z[38]=2.98, p=0.003; Z[38]=3.33, p=0.001, respectively). The results are illustrated in FIG. 3.

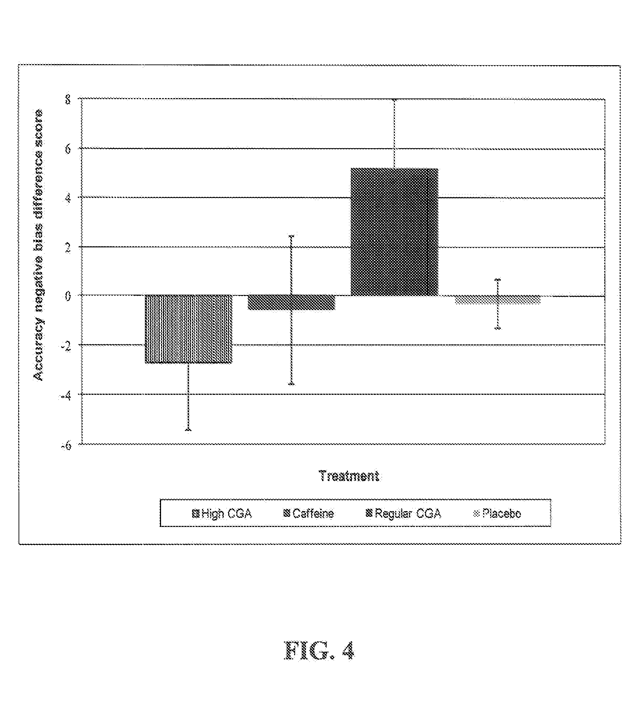

Finally, for the emotional processing behavioural task, parametric analyses revealed that high CGA content decaffeinated coffee reduced the negative bias accuracy index relative to placebo (trend level; F[1,38]=3.50, p=0.069) and the regular CGA content decaffeinated coffee (F[1,38]=6.08, p=0.018), which tended to increase the negative bias. FIG. 4 depicts the emotion face recognition task negative bias accuracy index difference score (with SEM error bars) for each treatment condition.

Together these mood results indicate that, like caffeine, decaffeinated coffee with higher total CGA content exerts some positive effects on mood processes. Specifically, it increased alertness and relaxation, and decreased the number of reports of headaches and mental fatigue relative to placebo and the regular CGA content decaffeinated coffee. Interestingly, the direction of results showed that the regular CGA content decaffeinated coffee resulted in increased headache and mental fatigue over time, suggesting that both caffeine and high CGA content decaffeinated coffee counteracted these effects. High CGA content decaffeinated coffee also affected the emotion face recognition task, reducing the negative bias accuracy index relative to regular CGA content decaffeinated coffee. This finding is somewhat in accord with the mood enhancing effects of high CGA content decaffeinated coffee and suggests that the high CGA content decaffeinated coffee may lead to a reduction in attentional 'negative bias' at a behavioral or higher-order level. Given that the high CGA content decaffeinated coffee contained only minute levels of caffeine, this suggests that some of the positive mood effects of coffee may relate to coffee compounds other than the caffeine itself.

### Conclusion

The present results provide the first indication that a high CGA content decaffeinated coffee, specifically, a decaffeinated coffee produced from a combination of unroasted and roasted coffee beans, exerts positive effects on sustained attention and mood. This indicates that there are compounds in coffee other than caffeine, most likely the chlorogenics acids, which have positive effects on mood and attention.

It should be understood that various changes and modifications to the presently preferred embodiments described herein will be apparent to those skilled in the art. Such changes and modifications can be made without departing from the spirit and scope of the present subject matter and without diminishing its intended advantages. It is therefore intended that such changes and modifications be covered by the appended claims.

## Claims

1. A method of sustaining attention in an individual, the method comprising:
administering to an individual in need of sustained attention a decaffeinated coffee product comprising an unroasted coffee to sustain attention in the individual.

2. The method of Claim 1, wherein the decaffeinated coffee product has a similar effect in the individual as an approximately equivalent amount of a caffeinated coffee product.

3. The method of any one of Claims 1 to 2, wherein the decaffeinated coffee product comprises a mixture of unroasted coffee and roasted coffee.

4. The method of any one of Claims 1 to 3, wherein the unroasted coffee comprises an extract of an unroasted coffee.

5. The method of Claim 4, wherein the extract is derived from:
(i) a first portion comprising unroasted ground and/or unground coffee, in an amount of from 1 to 90% by weight based on the total weight of the coffee product, and
(ii) a second portion comprising ground coffee that has been roasted to a higher degree of roast than the first portion, in an amount of from 99 to 10% by weight based on the total weight of the coffee product, wherein the content of the chlorogenic acids is at least 4 g per 100 g of the coffee product.

6. A method of improving an individual's mood or alertness, the method comprising:
administering to an individual in need improved mood or alertness a decaffeinated coffee product comprising an unroasted coffee to improve the mood or alertness in the individual.

7. The method of Claim 6, wherein the decaffeinated coffee product has a similar effect in the individual as an approximately equivalent amount of a caffeinated coffee product.

8. The method of any one of Claims 6 to 7, wherein the decaffeinated coffee product comprises a mixture of unroasted coffee and roasted coffee.

9. The method of any one of Claims 6 to 8, wherein the unroasted coffee comprises an extract of an unroasted coffee.

10. The method of Claim 9, wherein the extract is derived from:
(i) a first portion comprising unroasted ground and/or unground coffee, in an amount of from 1 to 90% by weight based on the total weight of the coffee product, and
(ii) a second portion comprising ground coffee that has been roasted to a higher degree of roast than the first portion, in an amount of from 99 to 10% by weight based on the total weight of the coffee product, wherein the content of the chlorogenic acids is at least 4 g per 100 g of the coffee product.

11. A method of improving relaxation in an individual, the method comprising:
administering to an individual in need of relaxation a decaffeinated coffee product comprising an unroasted coffee to improve relaxation in the individual.

12. The method of Claim 11, wherein the decaffeinated coffee product has a similar effect in the individual as an approximately equivalent amount of a caffeinated coffee product.

13. The method of any one of Claims 11 to 12, wherein the decaffeinated coffee product comprises a mixture of unroasted coffee and roasted coffee.

14. The method of any one of Claims 11 to 13, wherein the unroasted coffee comprises an extract of an unroasted coffee.

15. The method of Claim 14, wherein the extract is derived from:
(i) a first portion comprising unroasted ground and/or unground coffee, in an amount of from 1 to 90% by weight based on the total weight of the coffee product, and
(ii) a second portion comprising ground coffee that has been roasted to a higher degree of roast than the first portion, in an amount of from 99 to 10% by weight based on the total weight of the coffee product, wherein the content of the chlorogenic acids is at least 4 g per 100 g of the coffee product.

16. A method of reducing a headache in an individual, the method comprising:
administering to an individual having a headache a decaffeinated coffee product comprising an unroasted coffee to reduce the headache in the individual.

17. The method of Claim 16, wherein the decaffeinated coffee product has a similar effect in the individual as an approximately equivalent amount of a caffeinated coffee product.

18. The method of any one of Claims 16 to 17, wherein the decaffeinated coffee product comprises a mixture of unroasted coffee and roasted coffee.

19. The method of any one of Claims 16 to 18, wherein the unroasted coffee comprises an extract of an unroasted coffee.

20. The method of Claim 19, wherein the extract is derived from:
(i) a first portion comprising unroasted ground and/or unground coffee, in an amount of from 1 to 90% by weight based on the total weight of the coffee product, and
(ii) a second portion comprising ground coffee that has been roasted to a higher degree of roast than the first portion, in an amount of from 99 to 10% by weight based on the total weight of the coffee product, wherein the content of the chlorogenic acids is at least 4 g per 100 g of the coffee product.

21. A method of sustaining attention in an individual, the method comprising:
administering to an individual in need of sustained attention a non-coffee product comprising a component selected from the group consisting of an unroasted coffee extract, chlorogenic acids and combinations thereof to sustain attention in the individual.

22. The method of Claim 21, wherein the chlorogenic acids are derived from a food source.

23. A method of improving an individual's mood or alertness, the method comprising:
administering to an individual in need of an improved mood or alertness a non-coffee product comprising a component selected from the group consisting of an unroasted coffee extract, chlorogenic acids and combinations thereof to improve the mood or alertness of the individual.

24. The method of Claim 23, wherein the chlorogenic acids are derived from a food source.

25. A method of improving relaxation in an individual, the method comprising:
administering to an individual in need of an improved mood or alertness a non-coffee product comprising a component selected from the group consisting of an unroasted coffee extract, chlorogenic acids and combinations thereof to improve the relaxation of the individual.

26. The method of Claim 25, wherein the chlorogenic acids are derived from a food source.

27. A method of reducing a headache in an individual, the method comprising:
administering to an individual having a headache a non-coffee product comprising a component selected from the group consisting of an unroasted coffee extract, chlorogenic acids and combinations thereof to reduce the headache of the individual.

28. The method of Claim 27, wherein the chlorogenic acids are derived from a food source.

29. A method of making a consumable product for improving an individual's mental capabilities, the method comprising:
providing an unroasted coffee extract for improving the individual's mental capabilities; and
adding the unroasted coffee extract to a consumable product.

30. The method of Claim 29, wherein the consumable product is selected from the group consisting of a food product, a beverage, a food supplement, a pet care product, a pharmaceutical product and combinations thereof.

31. The method of any one of Claims 29 to 30, wherein the mental capabilities are selected from the group consisting of alertness, mood, attention and combinations thereof.

32. A method of improving an individual's mental capabilities, the method comprising:
providing an extract from an unroasted coffee product;
adding the extract to a consumable product; and
administering to an individual in need thereof the consumable product comprising the extract.

33. The method of Claim 32, wherein the consumable product is selected from the group consisting of a food product, a beverage, a food supplement, a pet care product, a pharmaceutical product and combinations thereof.

34. The method of any one of Claims 32 to 33, wherein the mental capabilities are selected from the group consisting of alertness, mood, attention and combinations thereof.

35. A method of improving an individual's mental capabilities, the method comprising:
administering to an individual in need thereof a consumable product comprising chlorogenic acids at a rate of about 1 to about 150 mg chlorogenic acids/kg bodyweight per day.

36. A method of improving an individual's mental capabilities, the method comprising:
administering to an individual in need thereof a decaffeinated coffee product comprising chlorogenic acids at a rate of about 1 to about 150 mg chlorogenic acids/kg bodyweight per day.

37. The use of an unroasted coffee extract to improve the attention, mood, relaxation or alertness or reduce the headache of an individual.

38. The use of chlorogenic acids to improve the attention, mood, relaxation or alertness or reduce the headache of an individual.

39. A method of increasing sports performance in an individual, the method comprising:
administering to an individual in need of thereof a decaffeinated coffee product comprising an unroasted coffee to increase the sports performance in the individual.

40. A method of increasing sports performance in an individual, the method comprising:
administering to an individual in need of thereof a non-coffee product comprising a component selected from the group consisting of an unroasted coffee extract, chlorogenic acids and combinations thereof to increase the sports performance in the individual.
